# EUROPÄISCHE PATENTSCHRIFT

(11) **EP 0 682 879 B1**
(45) Veröffentlichungstag und Bekanntmachung des Hinweises auf die Patenterteilung: **08.11.2000**
(21) Anmeldenummer: 95107610.8
(22) Anmeldetag: 18.05.1995
(51) Int. Cl.: A23L 1/30, A61K 31/23, A61K 31/22, A23D 7/00

(54) **Diätetisches Lebensmittel mit mittelkettigen Fettsäuren und dessen Verwendung**
Dietetic food containing medium chain fatty acids and use thereof
Aliments diététiques avec acides gras à chaîne moyenne et leur application

(30) Priorität: 20.05.1994 DE 4417851
(43) Veröffentlichungstag der Anmeldung: 22.11.1995
(73) Patentinhaber: HEIRLER, Horst, D-82237 Wörthsee (DE)
(72) Erfinder: HEIRLER, Horst, D-82237 Wörthsee (DE)
(74) Vertreter: VOSSIUS & PARTNER

(56) Entgegenhaltungen:
- EP-A- 0 597 295
- WO-A-89/02275
- WO-A-90/12080
- WO-A-94/15464
- PATENT ABSTRACTS OF JAPAN vol. 016 no. 437 (C-0984) ,11.September 1992 & JP-A-04 152861 (SNOW BRAND MILK PROD CO LTD) 26.Mai 1992,
- DATABASE WPI Section Ch, Week 9305 Derwent Publications Ltd., London, GB; Class B05, AN 93-041721 & JP-A-04 368 325 ( KAO CORP) , 21.Dezember 1992
- DATABASE WPI Section Ch, Week 9345 Derwent Publications Ltd., London, GB; Class B05, AN 93-357133 & JP-A-05 262 645 ( NIPPON OILS & FATS CO LTD) , 12.Oktober 1993

## Beschreibung

Die vorliegende Erfindung betrifft ein diätetisches Lebensmittel mit mittelkettigen Fettsäuren und langkettigen essentiellen Fettsäuren, einschließlich α-Linolsäure und α-Linolensäure, sowie gegebenenfalls weiteren Zusatzstoffen und dessen Verwendung zur ernährungstherapeutischen Behandlung von Patienten mit Störungen der Resorption von Nährstoffen (insbesondere von Fett und fettlöslichen Vitaminen) bei Malabsorption, insbesondere Zöliakie.

Bei der Glutenenteropathie (Zöliakie, einheimische Sprue) handelt es sich um eine Erkrankung des Dünndarmkanals, bei der spezifische Unverträglichkeitserscheinungen, die durch Gluten (Klebereiweiß in Weizen, Roggen, Hafer, Gerste und Dinkel) ausgelöst wird. Die Krankheit beruht auf einer Immunreaktion gegen Gluten, wodurch es zu Störungen der Resorption (Malabsorption) von Nährstoffen mit zahlreichen Symptomen auch von seiten des Zentralnervensystems kommt. Die bisherige Behandlung besteht vor allem in der Einhaltung einer streng glutenfreien Kost. Dafür stehen bisher zahlreiche glutenfreie diätetische Lebensmittel zur Verfügung, die teilweise lebenslang von den Erkrankten verwendet werden müssen.

Bei der Glutenenteropathie sind die Schleimhautschäden des Dünndarms durch eine Zottenatrophie gekennzeichnet. Daraus folgt eine starke Verminderung der Absorptionsfläche und eine Beeinträchtigung der absorptiven Funktion der intestinalen Epithelzellen. Mehr oder weniger spielt bei Zöliakie und Sprue neben der Glutensensibilität die obenerwähnte Malabsorption der Lipide eine Rolle, d.h. eine mehr oder minder starke Beeinträchtigung der Fettresorption. Sie macht sich durch Steathorroe bemerkbar, bei der teilweise das unverdaute Fett auf den Faeces der Patienten sichtbar ist.

Unter der beeinträchtigten Fettresorption ist bei Zöliakie- und Spruekranken auch die Resorptionsquote der fettlöslichen Vitamine A, D, E und K reduziert. Zudem ist die Resorption von Folsäure und Vitamin B12 beeinträchtigt. Auch Calcium, Eisen, Mangan und Zink sind von der Resorptionsstörung betroffen. Bestimmte Folgeerscheinungen der Glutenenteropathie werden hierdurch verursacht (z.B. Osteoporose, Anaemie, periphere und zentrale neurologische Störungen).

Die schlechte Ausnutzung des Nahrungsfettes und im Magen-Darmkanal auftretende Spaltprodukte von nicht resorbiertem Fett beschleunigen die Darmpassage und verstärken durch Verkürzung der Verweildauer der Speisen im Darm nochmals die bereits bei Zöliakie und Sprue vorliegende geminderte Nutzung der Nahrungsbestandteile (Malabsorption).

Die am Beispiel der Zöliakie dargestellte Malabsorption des Nahrungsfettes spielt auch bei anderen Darmerkrankungen, z.B. Erkrankungen der Gallenwege mit Gallensäuremangel, Erkrankungen der Bauchspeicheldrüse mit Mangel an Pankreas-Lipase, Mukoviszidose (zystische Pankreasfibrose), Status nach Resektion des Magens, der Bauchspeicheldrüse oder des Dünndarms, Leberzirrhose mit Gallensäuremangel, Morbus Whipple (mit Fettresorptionsstörung wie bei Zöliakie), Lymphabflußstörungen (z.B. durch Tumore) und Fettstoffwechselstörung Typ I eine Rolle.

Zu den bekannten diätetischen Lebenssmitteln für Patienten mit Glutenenteropathien gehören glutenfreie Backwaren, Brote, Backmischungen zur Herstellung glutenfreier Brote und Gebäcke sowie von Teigwären. Diese diätetischen Lebensmittel lösen das Problem, die Ernährung glutenfrei zu halten. Häufig bleibt jedoch eine mehr oder weniger große Beeinträchtigung der Fettverdauung und Fettresorption bestehen. Obwohl schon seit langem ein Bedürfnis besteht, dieser Beeinträchtigung durch entsprechende diätetische Maßnahmen entgegen zu wirken, unterblieben allerdings bisher geeignete Lösungsansätze.

WO 90/12080 offenbart ein Lebensmittel, das aus kurzkettigen Triglyceriden besteht. Allerdings enthält dieses keine α-Linolensäure und auch der Anteil an mittelkettigen Fettsäuren unterscheidet sich deutlich von dem Anteil des erfindungsgemäßen diatetischen Lebensmittels. WO 89/02275 offenbart ein Lebensmittel zur Behandlung von Patienten mit schlechten Ernährungszustand. Dabei werden Gemische aus ω-3/mittelkettigen Triglyceriden verwendet. Dieses Lebensmittel enthält ebenfalls jedoch keine α-Linolensäure und darüberhinaus ist der Gehalt an mittelkettigen Fettsäuren deutlich geringer als in dem erfindungsgemäßen diätetischen Lebensmittel.

Der vorliegenden Erfindung liegt somit das technische Problem zugrunde, ein diätetisches Lebensmittel für Patienten mit Malabsorptionssyndromen (z.B. bei Glutenenteropathie) zu schaffen, das nicht nur glutenfrei ist, sondern auch die mit der Erkrankung einhergehende stark verminderte Absorption von Lipiden und weiteren Stoffen berücksichtigt.

Die Lösung dieses technischen Problems erfolgt durch die Bereitstellung der in den Ansprüchen gekennzeichneten Ausführungsformen.

Alle Prozentangaben beziehen sich, soweit nicht anders augegeben auf Gewichtsprozent.

Die erfindungsgemäße Lösung ergibt sich somit durch die Bereitstellung eines diätetischen Lebensmittels, das in der Fettphase 70 bis 90 % mittelkettige Fettsäuren mit einer bestimmten Mischung hochungesättigter langkettiger essentieller Fettsäuren, einschließlich α-Linolsäure und α-Linolensäure, enthält.

Essentielle Fettsäuren sind Bausteine von Zellmembranen (Strukturlipide), Bausteine membrangebundener Enzyme, Vorstufen von Mediatoren (z.B. Prostaglandine, Leukotriene, Immunglobuline), die im Körper aus essentiellen Fettsäuren hervorgehen und vielfältige Wirkungen ausüben. Aus essentiellen Fettsäuren im Körper metabolisierte Mediatoren beeinflussen Immunstatus, Kreislauffunktionen (Blutgefäßkontraktion, Blutgefäßerweiterung, Blutdruck), Fließeigenschaften des Blutes, verhalten von Thrombozyten und Erythrozyten im Blut (Agglutinationstendenz), glatte Muskulatur (Atemwege, Darm). Aus α-Linolsäure C18: 2 n 6 entsteht im Stoffwechsel γ-Linolensäure C18: 3 n 6 als Vorstufe z.B. entzündungshemmender Mediatoren. Aus α-Linolensäure C18: 3 n 3 entsteht im Stoffwechsel Eicosapentaensäure C20: 5 n 3 als Vorstufe von Mediatoren.

Essentielle Fettsäuren werden im Stoffwechsel durch das Enzym Δ-6-Desaturase umgesetzt. Um dieses Enzym konkurrieren essentielle Fettsäuren und ihre Homologe. Bevorzugt umgesetzt wird durch dieses Enzym das jeweils niedrigere Homolog. Bei Mangel an Δ-6-Desaturase können höhere Homologe nicht mehr umgesetzt werden.

Mittelkettige Fettsäuren werden intestinal auch ohne Anwesenheit von Lipasen und/oder Gallensäuren aus mittelkettigen Triglyzeriden herausgelöst und resorbiert. Nach Aufnahme von mittelkettigen Fettsäuren in die Mukosazelle werden sie via Pfortader der Leber zugeführt und dort direkt umgesetzt. Digestion und Resorption mittelkettiger Fettsäuren erfolgt im Gegensatz zu langkettigen gesättigten Fettsäuren leicht und rasch. Mittelkettige Fettsäuren beanspruchen im Gegensatz zu langkettigen Fettsäuren nicht die Lymphwege, da sie vom Darm aus direkt in das Blut aufgenommen werden (wichtig bei Erkrankungen mit Stau, Blockade oder Verschluß der Lymphwege).

Das erfindungsgemäße diätetische Lebensmittel erreicht zum ersten Mal eine gezielte Supplementation mittelkettiger und essentieller langkettiger Fettsäuren, aus denen für den Darm wichtige Metaboliten hervorgehen.

Es wurde außerdem überraschenderweise herausgefunden, daß durch das erfindungsgemäße diätetische Lebensmittel die Fettresorption gesteigert werden kann und eine Normalisierung der Faeces noch besser als mit mittelkettigen Fettsäuren allein zu erreichen ist.

Der Einsatz dieses diätetischen Lebensmittels bewirkt jedoch nicht nur eine Verbesserung der Fettresorption, was bei dem reduzierten Ernährungszustand der Patienten wichtig ist, sondern kann darüber hinaus als Schiene für die Resorption weiterer Nahrungsinhaltsstoffe wirksam sein, deren Aufnahme sich bei Zöliakie, Sprue, Pankreasinsuffizienz, Gallensäuremangel, Mukoviszidose oder nach Dünndarmresektion als nützlich erwiesen hat.

In einer bevorzugten Ausführungsform enthält das erfindungsgemäße diätetische Lebensmittel in der Fettphase außerdem γ-Linolensäure. γ-Linolensäure C18: 3 n 6 geht im menschlichen Stoffwechsel aus α-Linolsäure C18: 2 n 6 hervor und ist Vorstufe von Mediatoren, die spezielle Wirkungen im Organismus ausüben (z.B. Einflüsse auf Entzündungsprozesse und Immunstatus. Wird γ-Linolensäure mit dem diätetischen Lebensmittel in der Ernährung eines Kranken mit Malabsorptionssyndrom zugeführt, ist sie direkt zum Umsatz daraus hervorgehender Mediatoren verfügbar. Anderenfalls müßte erst mit Hilfe des Enzyms Δ-6-Desaturase aus α-Linolsäure γ-Linolensäure metabolisiert werden. Bestimmte Mediatoren aus γ-Linolensäure haben einen positiven Einfluß auf krankhafte Vorgänge, die sich in der Darmmukosa bei Zöliakie oder anderen entzündlichen Darmerkrankungen abspielen.

In einer weiteren bevorzugten Ausführungsform beträgt der Gehalt an α-Linolsäure 5,9 bis 13,4 %.

In einer weiteren bevorzugten Ausführungsform enthält das diätetische Lebensmittel 1,4 bis 6,5 % α-Linolensäure.

Vorzugsweise enthält das diätetische Lebensmittel 0,1 bis 2,5 % γ-Linolensäure.

In noch einer weiteren bevorzugten Ausführungsform beträgt der Gehalt an gesättigten, langkettigen Fettsäuren höchstens 4 %.

Die in einer Menge von 70 bis 90 % in der Fettphase des erfindungsgemäßen Lebensmittels enthaltenen mittelkettigen Fettsäuren sind vorzugsweise Caprylsäure und Caprinsäure aus mittelkettigen Triglyceriden.

Die in einer Menge von 0,9 bis 4 % in dem erfindungsgemäßen diätetischen Lebensmittel enthaltenen gesättigten, langkettigen Fettsäuren stammen vorzugsweise zu 0,5 bis 2,4 % aus Safloröl, zu 0,3 bis 1,1 % aus Leinöl und zu 0,1 bis 0,5 % aus einem Emulgator, beispielsweise einem Gemisch aus Mono-/Diglyceriden und Lecithin.

Die in einer Menge von 5,9 bis 13,4 % in dem erfindungsgemäßen diätetischen Lebensmittel enthaltene α-Linolsäure stammt vorzugsweise zu 5,5 bis 11 % aus Safloröl und zu 0,4 bis 2,4 % aus Leinöl.

Leinöl stellt ebenfalls eine bevorzugte Quelle für die in einer Menge von 1,4 bis 6,5 % in dem erfindungsgemäßen Lebensmittel enthaltene α-Linolensäure dar.

Eine Quelle für die in einer Menge von 0,1 bis 2,5 % im erfindungsgemäßen Lebensmittel enthaltene γ-Linolensäure ist vorzugsweise Borretsch.

Die in einer Menge von 1,7 bis 3,6 % in dem erfindungsgemäßen diätetischen Lebensmittel enthaltenen Ölsäuren stammen vorzugsweise zu 1,1 bis 1,5 % aus Safloröl und zu 0,6 bis 2,1 % aus Leinöl.

In einer besonders bevorzugten Ausführungsform hat die Fettphase des diätetischen Lebensmittels folgende Zusammensetzung:

| | |
|---|---|
| Mittelkettige Fettsäuren | 70 - 90 % |
| Gesättigte, langkettige Fettsäuren | 0,9 - 4,0 % |
| α-Linolsäure | 5,9 - 13,4 % |
| α-Linolensäure | 1,4 - 6,5 % |
| γ-Linolensäure | 0,1 - 2,5 % |
| Ölsäure | 1,7 - 3,6 % |

In einer noch mehr bevorzugten Ausführungsform hat die Fettphase des diätetischen Lebensmittels folgende Zusammensetzung:

| | |
|---|---|
| Mittelkettige Fettsäuren | 80 % |
| Gesättigte, langkettige Fettsäuren | 1,6 % |
| α-Linolsäure | 9 % |
| α-Linolensäure | 5,4 % |
| γ-Linolensäure | 1,8 % |
| Ölsäure | 2,2 % |

In einer weiteren bevorzugten Ausführungsform enthält das diätetische Lebensmittel zusätzlich zu den oben angegebenen Fettsäuren weiterhin Emulgatoren, fettlösliche Vitamine, β-Karotin und/oder Lecithin.

Als Emulgator kann beispielsweise ein Emulgator aus Mono- und Diglyceriden sowie Lecithin in einer Konzentration von 0,5 % verwendet werden.

In einer besonders bevorzugten Ausführungsform des diätetischen Lebensmittels enthält die Fettphase die Vitamine A, D und/oder E.

Vorzugsweise beträgt die Fettphase des diätetischen Lebensmittels 80 % und die Wasserphase 20 %, wobei in einer bevorzugten Ausführungsform die Wasserphase Vitamin C, Folsäure und/oder Vitamin B12 enthält.

Bevorzugte Vitaminmengen pro 100 g Fett in der Margarine sind 0 bis 2 mg Vitamin A, 0 bis 300 µg β-Carotin, 0 bis 50 µg Vitamin D, 0 bis 100 mg Gesamttocopherol, 0 bis 1 µg Vitamin B12, 0 bis 5 mg Folsäure und 0 bis 75 mg Vitamin C.

Folsäure ist beteiligt an Prozessen der Zellteilung und der Zellneubildung. Bedeutsam ist, daß Patienten mit Erkrankungen der Darmschleimhaut (insbesondere bei Zöliakie) einen besonders hohen Bedarf an Folsäure besitzen, der zur Enterozyten-Regeneration erforderlich ist. Zusätzlich ist der Bedarf an Folsäure erhöht, um hyperchromer, makrozytärer Anämie entgegenzuwirken.

Durch den Zusatz von Vitamin C verbessert sich die Calcium- und Eisenresorption.

Eine weitere bevorzugte Ausführungsform betrifft daher die Verwendung des erfindungsgemäßen diätetischen Lebensmittels zur zur Herstellung eines diätetischen lebensmittels zur ernährungstherapeutischen Behandlung des Malabsorptionssyndroms. Das Malabsorptionssyndrom steht insbesondere mit folgenden krankhaften Zuständen in Zusammenhang: Gallensäuremangel, Pankreasinsuffizienz, chronischer Pankreatitis, Lerberzirrhose, Magenresektion (Postgastrektomiesyndrom), mechanischem Verschluß der Gallenwege, Gallensteinen, Gallensäuren-Inaktivierung durch Medikamente, Zöliakie, Erwachsenen-Sprue, Mukoviszidose (zystische Pankreasfibrose), Hyperlipoproteinämie Typ I (Triglycerid-Lipasemangel), Morbus Whipple oder Lymphabflußstörungen.

### Beispiel

### Diätetisches Lebensmittel in Form von Margarine

Das erfindungsgemäße diätetische Lebensmittel wird zweckmäßigerweise aufgrund seiner Hauptbestandteile bevorzugt in Form einer Margarine in den Verkehr gebracht.

Als Margarine enthält das erfindungsgemäße diätetische Lebensmittel ca. 18 Gew.-% Wasser, mindestens 80 Gew.-% Fett und ca. 2 Gew.-% Trockensubstanz. Ca. 20 % stellen die Wasserphase und ca. 80 % die Fettphase dar.

### Herstellung

1. Die wasserlöslichen Komponenten (Vitamin B12, Vitamin C, Folsäure, Aroma) werden im Wasser gelöst und vermischt. Anschließend wird auf 60 - 80°C erwärmt.
2. Die Fettkomponenten (mittelkettige Fettsäuren, Safloröl, Leinöl und Boretschöl) werden geschmolzen und vermischt.
3. 1 Teil Emulgator wird mit 5 Teilen "Fettkomposition" von Schritt 2 auf 65°C erwärmt und klar geschmolzen, dann diese Mischung zur gesamten "Fettkomposition" gegeben und vermischt.
4. Fettlösliche Komponenten (Vit. E, Vit. D 3, Vit. A-Palmitat, β-Carotin) werden darin aufgelöst und vermischt.
5. Fettphase und Wasserphase werden bei 40 bis 50°C unter Rühren so gemischt, daß eine Emulsion vom Typ W/O (Wasser in Öl) gebildet wird.
6. Die entstandene W/O-Emulsion wird in einem Schabewärmeaustauscher (Rohrkühler) in der für Margarine bekannten Weise kristallisiert und geknetet, so daß ein streichfähiges Produkt erhalten wird.

Der Tagesbedarf der Diätmargarine ist variabel, muß sich jedoch nach dem jeweiligen Befund richten. Bei guter Verträglichkeit können 50 bis 70 g Diätmargarine pro Tag von dem Patienten zu sich genommen werden.

## Patentansprüche

1. Diätetisches Lebensmittel enthaltend in der Fettphase:
(a) 70 bis 90 Gew.-% mittelkettige Fettsäuren,
(b) α-Linolsäure und
(c) α-Linolensäure.

2. Diätetisches Lebensmittel nach Anspruch 1, das in der Fettphase weiterhin γ-Linolensäure enthält.

3. Diätetisches Lebensmittel nach Anspruch 1 oder 2, wobei der Gehalt an α-Linolsäure 5,9 bis 13,4 Gew.-% ist.

4. Diätetisches Lebensmittel nach einem der Ansprüche 1 bis 3, wobei der Gehalt an α-Linolensäure 1,4 bis 6,5 Gew.-% ist.

5. Diätetisches Lebensmittel nach einem der Ansprüche 1 bis 4, wobei der Gehalt an γ-Linolensäure 0,1 bis 2,5 Gew.-% ist.

6. Diätetisches Lebensmittel nach einem der Ansprüche 1 bis 5, wobei der Gehalt an gesättigten, langkettigen Fettsäuren höchstens 4 Gew.-% beträgt.

7. Diätetisches Lebensmittel nach einem der Ansprüche 1 bis 6, wobei die Fettphase folgende Zusammensetzung hat:
| | |
|---|---|
| Mittelkettige Fettsäuren | 70 - 90 Gew.-%, |
| Gesättigte, langkettige Fettsäuren | 0,9 - 4,0 Gew.-%, |
| α-Linolsäure | 5,9 - 13,4 Gew.-%, |
| α-Linolensäure | 1,4 - 6,5 Gew.-%, |
| γ-Linolensäure | 0,1 - 2,5 Gew.-%, |
| Ölsäure | 1,7 - 3,6 Gew.-%. |

8. Diätetisches Lebensmittel nach einem der Ansprüche 1 bis 7, wobei die Fettphase folgende Zusammensetzung hat:
| | |
|---|---|
| Mittelkettige Fettsäuren | 80 Gew.-%, |
| Gesättigte langkettige Fettsäuren | 1,6 Gew.-%, |
| α-Linolsäure | 9 Gew.-%, |
| α-Linolensäure | 5,4 Gew.-%, |
| γ-Linolensäure | 1,8 Gew.-%, |
| Ölsäure | 2,2 Gew.-%. |

9. Diätetisches Lebensmittel nach einem der Ansprüche 1 bis 8, wobei die Fettphase außerdem Emulgatoren, fettlösliche Vitamine, β-Karotin und/oder Lecithin enthält.

10. Diätetisches Lebensmittel nach Anspruch 9, wobei die fettlöslichen Vitamine die Vitamine A, D und/oder E sind.

11. Diätetisches Lebensmittel nach Anspruch 1 bis 10, wobei die Fettphase 80 Gew.-% und die Wasserphase 20 Gew.-% sind.

12. Diätetisches Lebensmittel nach Anspruch 11, wobei die Wasserphase Vitamin C, Folsäure und/oder Vitamin B12 enthält.

13. Verwendung der in einem der Ansprüche 1 bis 12 definierten Zusammensetzung zur Herstellung eines diätetischen Lebensmittels zur ernährungstherapeutischen Behandlung des Malabsorptionssyndroms.

14. Verwendung nach Anspruch 13, wobei das Malabsorptionssyndrom im Zusammenhang mit Gallensäuremangel, Pankreasinsuffizienz, chronischer Pankreatitis, Leberzirrhose, Magenresektion (Postgastrektomiesyndrom), mechanischem Verschluß der Gallenwege, Gallensteinen, Gallensäuren-Inaktivierung durch Medikamente, Zöliakie, Erwachsenen-Sprue, Mukoviszidose (zystische Pankreasfibrose), Hyperlipoproteinämie Typ I (Triglycerid-Lipasemangel), Morbus Whipple oder Lymphabflußstörungen steht.

## Claims

1. Dietary food containing in fat phase:
(a) 70 - 90 % w/v medium-chain fatty acids
(b) α-linolic acid and
(c) α-linolenic acid.

2. Dietary food according to claim 1 further containing γ-linolenic acid in fat phase.

3. Dietary food according to claim 1 or 2 wherein the content of α-linolic acid is 5.9 to 13.4% w/v.

4. Dietary food according to any one of claims 1 to 3 wherein the content of α-linolenic acid is 1.4 to 6.5 % w/v.

5. Dietary food according to any one of claims 1 to 4 wherein the content of γ-linolenic acid is 0.1 to 2.5 % w/v.

6. Dietary food according to any one of claims 1 to 5 wherein the content of saturated long-chain fatty acids is 4 % w/v at the most.

7. Dietary food according to any one of claims 1 to 6 wherein the fat phase contains the following:
| | |
|---|---|
| Medium-chain fatty acids | 70 - 90 % w/v |
| Saturated long-chain fatty acids | 0.9 - 4.0 % w/v |
| α-linolic acid | 5.9 - 13.4 % w/v |
| α-linolenic acid | 1.4 - 6.5 % w/v |
| γ-linolenic acid | 0.1 - 2.5 % w/v |
| Oleic acid | 1.7 - 3.6 % w/v |

8. Dietary food according to any one of claims 1 to 7 wherein the fat phase contains the following:
| | |
|---|---|
| Medium-chain fatty acids | 80 % w/v |
| Saturated long-chain fatty acids | 1.6 % w/v |
| α-linolic acid | 9 % w/v |
| α-linolenic acid | 5.4 % w/v |
| γ-linolenic acid | 1.8 % w/v |
| Oleic acid | 2.2 % w/v |

9. Dietary food according to any one of claims 1 to 8 wherein the fat phase also contains emulsifiers, fat-soluble vitamins, β-carotene, and/or lecithin.

10. Dietary food according to claim 9 wherein the fat-soluble vitamins are the vitamins A, D and/or E.

11. Dietary food according to any one of claims 1 to 10 wherein the fat phase is 80 % w/v and the water phase 20 % w/v.

12. Dietary food according to claim 11 wherein the water phase contains vitamin C, folic acid and/or vitamin B12.

13. Use of any one of the combinations defined in claims 1 to 12 for the production of dietary food in dietotherapeutic treatment of malabsorption syndrome.

14. Use according to claim 13 whereby the malabsorption syndrome is linked with lack of bile acids, pancreatic insufficiency, chronic inflammation of the pancreas, liver cirrhosis, gastric resection (postgastrectomy syndrome), mechanical closure of the bile ducts, biliary stones, inactivity of the bile acids due to drugs, celiac disease, adult psilosis, cystic fibrosis (cystic fibrosis of the pancreas), hyperlipoproteinemia type I (triacylglycerol lipase deficiency), Morbus Whipple or deficiency of lymphatic drainage.

## Revendications

1. Aliment diététique contenant dans la phase grasse :
(a) de 70 à 90 % en poids d'acides gras à moyenne longueur de chaîne,
(b) de l'acide α-linoléique, et
(c) de l'acide α-linolénique.

2. Aliment diététique selon la revendication 1, qui dans la phase grasse contient en outre de l'acide γ-linolénique.

3. Aliment diététique selon la revendication 1 ou 2, dont la teneur en acide α-linoléique est de 5,9 à 13,4 % en poids.

4. Aliment diététique selon l'une des revendications 1 à 3, dont la teneur en acide α-linolénique est de 1,4 à 6,5 % en poids.

5. Aliment diététique selon l'une des revendications 1 à 4, dont la teneur en acide γ-linolénique est de 0,1 à 2,5 % en poids.

6. Aliment diététique selon l'une des revendications 1 à 5, dont la teneur en acides gras saturés à longue chaîne est d'au plus 4 % en poids.

7. Aliment diététique selon l'une des revendications 1 à 6, dont la phase grasse a la composition suivante :
| | |
|---|---|
| Acides gras à moyenne longueur de chaîne | 70-90 % en poids |
| Acides gras saturés à longue chaîne | 0,9-4,0 % en poids |
| Acide α-linoléique | 5,9-13,4 % en poids |
| Acide α-linolénique | 1,4-6,5 % en poids |
| Acide γ-linolénique | 0,1-2,5 % en poids |
| Acide oléique | 1,7-3,5 % en poids |

8. Aliment diététique selon l'une des revendications 1 à 7, dont la phase grasse a la composition suivante :
| | |
|---|---|
| Acides gras à moyenne longueur de chaîne | 80 % en poids |
| Acides gras saturés à longue chaîne | 1,6 % en poids |
| Acide α-linoléique | 9 % en poids |
| Acide α-linolénique | 5,4 % en poids |
| Acide γ-linolénique | 1,8 % en poids |
| Acide oléique | 2,2 % en poids |

9. Aliment diététique selon l'une des revendications 1 à 8, dont la phase grasse contient en outre des émulsifiants, des vitamines liposolubles, du β-carotène et/ou de la lécithine.

10. Aliment diététique selon la revendication 9, dont les vitamines liposolubles sont les vitamines A, D et/ou E.

11. Aliment diététique selon les revendications 1 à 10, dont la phase grasse représente 80 % en poids et la phase aqueuse 20 % en poids.

12. Aliment diététique selon la revendication 11, dont la phase aqueuse contient de la vitamine C, de l'acide folique et/ou de la vitamine B12.

13. Utilisation de la composition définie dans l'une des revendications 1 à 12 pour préparer un aliment diététique destiné au traitement par thérapie nutritionnelle du syndrome de malabsorption.

14. Utilisation selon la revendication 13, pour laquelle le syndrome de malabsorption est lié à une carence en acide gallique, une insuffisance pancréatique, une pancréatite chronique, une cirrhose du foie, une résection partielle de l'estomac (syndrome de chasse), une oblitération mécanique des voies biliaires, des calculs biliaires, une inactivation de l'acide gallique par des médicaments, une maladie coeliaque, une sprue de l'âge adulte, une mucoviscidose (fibrose kystique du pancréas), une hyperlipoprotéinémie de type 1 (carence en triglycéride-lipases), une lipodystrophie intestinale ou des troubles de l'écoulement lymphatique.
